# EUROPEAN PATENT APPLICATION

(11) **EP 2 292 281 A1**
(43) Date of publication of application: **09.03.2011**
(21) Application number: 09769371.7
(22) Date of filing: 27.04.2009
(51) Int. Cl.: A61M 1/00, A61F 5/453, A61F 5/455

(54) **ELECTROMECHANICAL ASSEMBLY FOR TREATING THE URINARY SYSTEM**

(30) Priority: 23.06.2008 ES 200801868
(71) Applicant: Vázquez Torruella, Jaime, 28005 Madrid (ES)
(72) Inventor: Vázquez Torruella, Jaime, 28005 Madrid (ES)
(74) Representative: Isern-Jara, Nuria
(86) International application number: PCT/ES2009/000227
(87) International publication number: WO 2009/156525

(57) **Abstract**

The invention relates to an electromechanical assembly for treating the urinary system, comprising a urethrovesical probe for extracting material and/or administering nected to the urethrovesical probe, said pumping means being associated with a programming and timing device which controls the frequency and flowrate of the material to be extracted via the urethrovesical probe from the treated zone and/or the substances to be administered into the urethra; and storage means for storing the material to be extracted and/or the substances to be administered. This results in a versatile assembly which prevents possible obstruction of the probe channels since the material is transported in a controlled and automated manner at all times.

## Description

### OBJECT OF THE INVENTION

The present invention refers to an electromechanical assembly provided with a urethrovesical probe for treating the urinary system that incorporates significant innovations and advantages when compared to other probes and devices of the prior art.

### BACKGROUND OF THE INVENTION

The use of surgical probes for treating the urinary system, such as, for example, those described in the utility model number 0244034 has been well-known for years.

These probes carry out the evacuation of liquids and vesical residues through three orifices located in the upper end of the probe and with an outlet located at the bottom end. These residues are extracted by gravity. Usually, in the majority of cases it is necessary to unblock the probe of residues produced by vesical surgery, clots, etc., with the corresponding inconveniences that these may cause in the patient.

Those probes provided with a balloon approximately 30 mm. in diameter, oval in shape and which is usually located approximately 80 mm. from the upper end of the probe are also known. This balloon is supported on the inside neck of the bladder between the inner sphincter and the prostate. Nonetheless, if the intervention is carried out in the neck of the bladder, inner sphincter or prostate, the balloon body is recognized by the organism as a foreign body, causing unwanted side effects in the patient, such as a spasmodic crisis.

### DESCRIPTION OF THE INVENTION

The invention herein has been developed with the aim of providing an electromechanical assembly for treating the urinary system that resolves the aforementioned drawbacks, also providing other additional advantages that will become apparent from the description provided hereinafter.

It is therefore an object of the invention to provide an electromechanical assembly for treating the urinary system, which comprises a urethrovesical probe, such as the type that has two to five access paths and is essentially characterized in that the operating system uses pumping, absorption or injection means, attached to a programming and timing device, such that it controls different frequencies, flow and substances to be inserted or removed from the inside of the bladder and the urethra and the storage means for said devices and substances.

In one embodiment of the invention, these storage means may consist of separate containers, with one of them for the storage of material from the urethra while the other is filled with a fluid to be dispensed on the inside of the urethra.

The pumping means consists of at least one electropump, having arranged an electropump connected to the storage means in order to store the material from the urethra and at least one electropump connected to the fluid supply means.

Thanks to these characteristics, a versatile assembly is obtained that prevents the possible obstruction of the probe pathways since at all times the transport of the material is carried out in a controlled and automated manner. Additionally, the fact that there are programming means attached to the supply means allow for dispensing according to the patient's needs the fluids or substances, such as lubricants and antispasmodics, that need to be provided to the inside of the bladder in a precise and safe manner.

According to another aspect of the invention, the pumping means are connected to the urethrovesical probe through an elongated, flexible tube.

Advantageously, it comprises self-contained power supply means associated to the programming and timing device that provide autonomy to the assembly described hereinabove, making it possible for the patient to be able to move and leave the hospital, bringing with him/her the assembly, previously adapted in weight and dimensions, comfortably and easily on being protected from incontinence and other urinary retention or any other urological event.

Also advantageously, the urethrovesical probe of the invention includes a self-retaining ring arranged around the flexible tube, which during its application in the urinary system is supported by the walls of the bladder without touching the neck thereof such that it does not put pressure on the bladder neck, inner sphincter and prostate. In this way the urethrovesical probe is provided with a firm hold on the inside of the bladder.

Additionally, the electromechanical assembly comprises an electric motor attached to an elongated helicoidal axis that passes through the interior of the flexible tube in order to extract all types of solid surgical residues from the inside of the bladder that acts as a drainage element.

Other characteristics and advantages of the assembly object of the present invention will become apparent from the description of a preferred, although not exclusive embodiment, that is illustrated by way of non-limiting example in the drawings appended, in which:

### BRIEF DESCRIPTION OF THE DRAWINGS

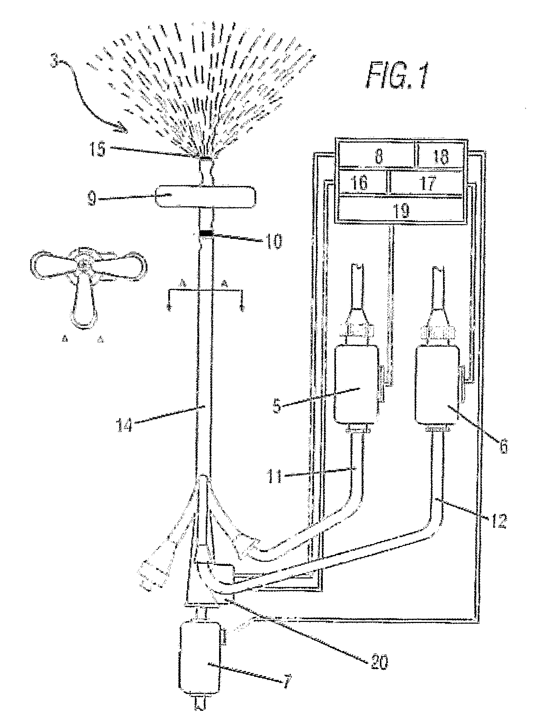

Figure 1.- Schematic view of an electromechanical assembly for treating the urinary system in accordance with the present invention which includes a sectional detail along line A-A; and

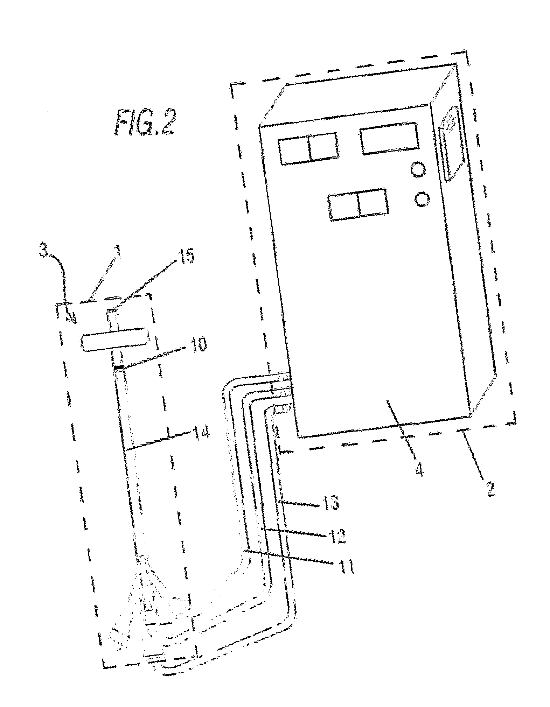

Figure 2.- Schematic view of the above shown assembly.

### DESCRIPTION OF A PREFERRED EMBODIMENT

An easy to use and transportable electromechanical assembly for treating the urinary system, especially for those patients with acute urine retention due to prostatism, formation of urethrovesical clots, extravasation of urine, transurethral resection, essentially comprises two clearly distinct parts shown graphically in two rectangles numbered 1 and 2 which are explained hereinafter.

One of the parts (1) corresponds to a urethrovesical probe (3) which will be explained below, whilst the second part corresponds to a series of mechanical-electrical and electronic components housed on the inside of the housing body (4) of plastic material between which there is a plurality of electropumps (5), (6) and (7) connected to a urethrovesical probe (3), while said electropumps (5), (6) and (7) are associated to a corresponding programming and timing device (8), (17) and (18) that can be handled by an expert such that the frequency and flow of the captured material (residues, urine) from the urethrovesical probe (3) from the treated area as well as the fluids to be inserted into the urethra, and storage means for storing said material and substances to be supplied. It should be mentioned that the housing body (4) is of the portable type such that it improves the quality of life of patientes to be treated by means of the use of the urethrovesical probe (3) of the invention. The programming and timing device is formed by three electronic assemblies (8), (17) and (18) which may or may not be interconnected to each other.

Said storage means consists of three hermetically sealed containers (not shown), with one of them being for the storage of the material from the urethra whilst the other two, with a capacity of 500 ml, are filled with a fluid to be administered by injection into the inside of the urethra, which are intended for the washing, instillation and irrigation of the urinary bladder. No further detail will be given about the programming-timing device (8) and any type of electronic device with programming and timer control functions commercially available can be used.

Additionally, means are provided for self-contained power supply associated to the programming-timing device consisting of a 12V battery which provide energy autonomy to the assembly described such that the patient can take it with him/her.

From each one of the separate containers, a conduit protrudes (11), (12) and (13) that circulates through the inside of the flexible tube (14) towards the upper end of the urethrovesical probe, with each one of the conduits attached to the corresponding electropump thereof (5), (6) and (7), respectively. The conduit for the evacuation of the material or substances to be removed includes an elongated helicoidal axis on the inside (not shown) attached to an electric motor that passes through to extract the material coming from the inside of the bladder.

The urethrovesical probe (3) provided with inlet and outlet orifices for the extraction and supply of substances includes a self-retaining ring (9) with a diameter ranging between 30-50 mm and 10 mm thick arranged around and on the upper part of the flexible tube (14). Below said self-retaining ring (9) a sensor (10) connected to an electronic processor (16) is provided to detect the formation of electrical signals on the inside of the urinary system, in particular the electric shocks emitted on irritating the receptors sensitive to existing stretching or compression on the walls of the urethral organ when the probe is inserted. In addition, at the upper end of the urethrovesical probe (3) it is provided with a spray valve (15) which improves the distribution of substances to be supplied on the inside of the patient's bladder.

The assembly described in the memory herein is suitable for treating patients with a neurogenic bladder, the administration of endovesical therapies, surgery postoperative, such as urethra, prostate and bladder, vesical fistulas and extraperitoneal bladder rupture, vesicoprostatic hematuria in patients requiring vesical washing, infravesical obstruction or vesical atony.

The details, shapes, dimensions and other accessory elements, as well as the materials used in the manufacture of the electromechanical assembly for treating the urinary system of the invention may be conveniently replaced by others which are technically equivalent and do not depart from the essential nature of the invention or from the scope defined by the claims appended hereinafter.

## Claims

1. An electromechanical assembly for treating the urinary system, comprising a urethrovesical probe for the extraction of material and the supply of substances to the inside of the urinary system, **characterized in that** it comprises pumping means connected to the urethrovesical probe, with said pumping means being associated to a programming-timing device such that it controls the frequency and flow of the material to be extracted from the urethrovesical probe from the treated area and/or substances to be supplied within the urethra, and storage means for storing said material to be extracted and substances to be supplied.

2. An electromechanical assembly according to claim 1, **characterized in that** the pumping means are connected to the urethrovesical probe through an elongated, flexible tube.

3. An electromechanical assembly according to claim 1, **characterized in that** it comprises self-contained power supply means associated to the programming-timing device and the pumping means.

4. An electromechanical assembly according to claim 3, **characterized in that** such power supply means consists of a battery.

5. An electromechanical assembly according to claim 1, **characterized in that** the urethrovesical probe includes a self-retaining ring arranged around and on the upper part of the flexible tube.

6. An electromechanical assembly according to claims 1 and 2, **characterized in that** it comprises an electric motor attached to an elongated helicoidal axis that passes through the interior of the flexible tube in order to extract the material from the inside of the bladder.

7. An electromechanical assembly according to claim 1, **characterized in that** the storage means comprises separate containers, with at least one of them being intended to the storage of material from the urethra, while at least one other container is filled with a fluid to be dispensed on the inside of the urethra.

8. An electromechanical assembly according to claim 1, **characterized in that** the storage means and the pumping means are housed on the inside of the housing body.

9. An electromechanical assembly according to claim 1, **characterized in that** the pumping means consists of at least one electropump.

10. An electromechanical assembly according to claims 1 and 9, **characterized in that** an electropump is connected to the storage means for storing the material coming from the urethra.

11. An electromechanical assembly according to claims 1 and 9, **characterized in that** at least one electropump is connected to the fluid supply means.

12. An electromechanical assembly according to claim 1, **characterized in that** the urethrovesical probe includes a sensor to detect the formation of electrical signals on the inside of the urinary system.

13. An electromechanical assembly according to claim 7, **characterized in that** from each one of the separate containers a conduit protrudes that circulates through the inside of the flexible tube to the upper end of the urethrovesical probe, each one of the conduits being associated to the pumping means.

14. An electromechanical assembly according to claim 1, **characterized in that** the urethrovesical probe includes a spray valve on the upper end thereof.

15. An electromechanical assembly according to claims 9, 10 or 11, **characterized in that** each electropump is associated to an individual programming-timing device.
